# EUROPÄISCHE PATENTSCHRIFT

(11) **EP 0 763 367 B1**
(45) Veröffentlichungstag und Bekanntmachung des Hinweises auf die Patenterteilung: **02.07.2003**
(21) Anmeldenummer: 96114208.0
(22) Anmeldetag: 05.09.1996
(51) Int. Cl.: A61M 1/34

(54) **Verfahren zum Überprüfen von mindestens einem, in einem Dialysierflüssigkeitssystem angeordneten Filter**
Method for testing at least one filter mounted in a dialyse circuit
Méthode pour tester au moins un filtre monté dans un circuit de dialyse

(30) Priorität: 16.09.1995 DE 19534417
(43) Veröffentlichungstag der Anmeldung: 19.03.1997
(73) Patentinhaber: Fresenius Medical Care Deutschland GmbH, 61352 Bad Homburg v.d.H. (DE)
(72) Erfinder: Wamsiedler, Ralf, 97469 Gochsheim-Weyher (DE); Mathieu, Bernd, Dr., 66583 Spiesen (DE)
(74) Vertreter: Luderschmidt, Schüler & Partner GbR

(56) Entgegenhaltungen:
- EP-A- 0 491 981
- DE-C- 3 448 262
- DE-U- 8 305 713
- US-A- 4 614 109
- US-A- 4 872 974

## Beschreibung

Die Erfindung betrifft ein Verfahren zum Überprüfen von mindestens einem im Dialysierflüssigkeitssystem einer Vorrichtung zur extrakorporalen Blutbehandlung angeordneten Filter, der durch eine Keime zurückhaltende Membran in eine erste Kammer und eine zweite Kammer geteilt ist gemäß dem Oberbegriff des Patentanspruchs 1. Ferner betrifft die Erfindung ein Dialysierflüssigkeitssystem einer Vorrichtung zur extrakorporalen Blutbehandlung, das mindestens einen Filter enthält, der durch eine Keime zurückhaltende Membran in eine erste Kammer und eine zweite Kammer geteilt ist, gemäß dem Oberbegriff des Patentanspruchs 5.

Bei der Hämofiltration wird - ähnlich wie bei der Hämodialyse - Blut an der Membran eines Hämofilters vorbeigeleitet, wobei ein Teil des Serums durch die Membran abgezogen und durch eine sterile Substitutionsflüssigkeit ersetzt wird, die entweder stromauf des Dialysators (Prädilution) oder stromab des Dialysators (Postdilution) dem extrakorporalen Blutweg zugesetzt wird. Zusätzlich wird bei der Hämodiafiltration noch die übliche Hämodialyse durchgeführt, d.h. es wird an der Membran des Hämodialysators Dialysierflüssigkeit vorbeigeleitet, so daß über die Membran hinweg ein Austausch von harnpflichtigen Substanzen erfolgen kann.

Die Dialysierflüssigkeit kann on-line aus Frischwasser und einem Elektrolytkonzentrat und die Substitutionsflüssigkeit on-line aus der Dialysierflüssigkeit hergestellt werden. Das Elektrolytkonzentrat ist zwar in der Regel steril und das Frischwasser weist üblicherweise keine Keime auf, es ist jedoch nicht sichergestellt, daß die on-line hergestellte Dialysierflüssigkeit absolut steril und pyrogenfrei ist, weshalb die Dialysierflüssigkeit zur Herstellung der Substitutionsflüssigkeit in den sterilen und pyrogenfreien Zustand überführt wird. Dazu wird stromauf des Dialysators Dialysierflüssigkeit entnommen, welche durch mindestens einen Filter geleitet wird, der durch eine Keime zurückhaltende hydrophile Membran in zwei Kammern geteilt ist. Eine derartige Vorrichtung mit zwei im Dialysierflüssigkeitssystem angeordneten Filtern ist beispielsweise aus DE 34 44 671 C2 bekannt.

Zum Überprüfen der Dichtheit der Filter der aus DE 34 44 671 C2 bekannten Hämo(dia)filtrationsvorrichtung wird die im Dialysierflüssigkeitskreislauf angeordnete Ultrafiltrationspumpe in Betrieb genommen, die über ein hydrophobes, mikroporöses Filter Luft in jeweils eine der beiden Kammern der Filter fördert. Da die Membranen der beiden Filter durch den der Integritätsprüfung vorausgehenden Spülvorgang bereits mit einer wässrigen Lösung benetzt sind, kann die eingeströmte Luft nicht über die Membranen der Filter entweichen, so daß sich die Membranen auf etwaige Lecks mittels des Unterdrucks überprüfen lassen, der durch die Ultrafiltrationspumpe erzeugt wird. Die Ultrafiltrationspumpe wird so lange betrieben, bis sich ein bestimmter Unterdruck im flüssigkeitsgefüllten Teil des Dialysierflüssigkeitssystems aufgebaut hat. Anschließend wird die Änderung des Unterdrucks an einem Druckmeßgerät überwacht und die Zeit gemessen, die der Druck braucht, um auf einen bestimmten Wert gegen Atmosphäre anzusteigen. Auf eine Undichtigkeit wird dann geschlossen, wenn eine bestimmte Zeitdauer überschritten wird. Es ist aber auch möglich, nach einer vorgegebenen Zeit den erreichten Unterdruck auszuwerten (forward flow test). Ein derartiges Verfahren zur Überprüfung der Dichtheit der Filter bei der aus DE 34 44 671 C2 bekannten Hämo(dia)filtrationsvorrichtung ist in DE 34 48 262 C2 beschrieben. Zwar hat sich der Unterdrucktest in der Praxis grundsätzlich bewährt, als nachteilig hat sich jedoch die Dauer der Testphase erwiesen. Für den Flüssigkeitsentzug werden bei dem bekannten Verfahren etwa 3 Minuten benötigt. Die gesamte Testzeit beträgt etwa 10 Minuten.

Die EP-A-0 491 981 beschreibt eine automatische Anlage zur Herstellung von Dialysekonzentraten. Die Überprüfung eines Filters, der Bestandteil der Anlage ist, erfolgt mittels eines Überdrucktests. Der Überdruck im Flüssigkeitssystem wird mittels einer Gaspumpe aufgebaut, die ansonsten zum Abpumpen des bei der Herstellung des Dialysekonzentrats in der Mischbehältereinheit entweichenden Gases Verwendung findet.

Verfahren und Vorrichtungen zum Prüfen von mit Flüssigkeit benetzten Membranen mittels eines Überdrucktests sind auch aus der US-A-4 872 974 und US-A-4 614 109 bekannt. Aus der DE-U-83 05 713 ist ein Gerät zur Aufbereitung medizinischer Infusionslösungen bekannt, das über einen Mischbehälter verfügt, auf dessen Auslass zur sterilen Belüftung und Entlüftung ein Belüftungsfilter aufgesetzt ist.

Der Erfindung liegt die Aufgabe zugrunde, ein Verfahren anzugeben, das eine schnelle und sichere Überprüfung von mindestens einem im Dialysierflüssigkeitssystem einer Vorrichtung zur extrakorporalen Blutbehandlung angeordneten Filter auf Lecks erlaubt. Ferner liegt der Erfindung die Aufgabe zugrunde, ein Dialysierflüssigkeitssystem einer Vorrichtung zur extrakorporalen Blutbehandlung derart auszubilden, dass eine schnelle und sichere Überprüfung der Dichtheit der Filter möglich ist.

Die Lösung der Aufgabe erfolgt erfmdungsgemäß mit den Merkmalen des Patentanspruchs 1 bzw. 5.

Zweckmäßige Weiterbildungen der Erfindung sind Gegenstand der betreffenden abhängigen Ansprüche.

Das erfindungsgemäße Verfahren beruht auf einem Überdrucktest. Bei dem erfindungsgemäßen Verfahren wird die Membran der Filter mit einer Wässrigen Flüssigkeit völlig benetzt und in den Zweig des Dialysierflüssigkeitssystems, der eine der beiden Kammern des Filters enthält, wird so lange Gas geleitet, bis ein vorbestimmter Überdruck in der Kammer aufgebaut ist. Dabei wird die zur völligen Benetzung der Filtermembran zuvor in die Kammer geleitete wässrige Lösung über die Filtermembran aus der Kammer verdrängt. Der Überdruck in der Kammer wird dann überwacht, um eine Aussage über die Membranintegrität treffen zu können. Für den Fall, dass der Druck innerhalb einer vorbestimmten Zeitdauer auf einen wert abgefallen ist, der kleiner ist als ein vorbestimmter Mindestdruck, wird auf eine Undichtigkeit der Membran geschlossen. Es kann aber auch die Zeitdauer gemessen werden, die der Druck braucht, um auf einen vorbestimmten Wert abzufallen.

Als wässrige Flüssigkeit zur Benetzung der Membran kann die im Dialysierflüssigkeitssystem während des Betriebs der Blutbehandlungsvorrichtung befindliche Dialysierflüssigkeit Verwendung finden. Eine Überprüfung der Filter kann sich z. B. in vorteilhafter Weise an die Spülphase der Blutbehandlungsvorrichtung anschließen, wenn die Filtermembran mit der Spüllösung völlig benetzt ist.

Beim Überprüfen der im Dialysierflüssigkeitssystem einer Vorrichtung zur extrakorporalen Blutbehandlung angeordneten Filter mittels Überdruck ergeben sich überraschenderweise eine Reihe von Vorteilen.

Zunächst lassen sich mit Überdruck wesentlich höhere Druckdifferenzen gegenüber der Atmosphäre erzeugen, so daß die Testempfindlichkeit entscheidend verbessert wird. Die Druckdifferenz kann in der Praxis bis zu 4 bar betragen, während der maximal mögliche Unterdruck im Dialysierflüssigkeitssystems theoretisch lediglich 1 bar betragen kann. Bei den bekannten Prüfverfahren liegt der in Teilen des Dialysierflüssigkeitssystems erzeugte Unterdruck sogar nur bei 0,3 bar. Mit dem erfindungsgemäßen Verfahren lassen sich auch sehr kleine Risse in der Membran dedektieren. Bezogen auf die Prüfung mit Unterdruck wird die Empfindlichkeit um das Drei- bis Vierfache erhöht.

Ferner ist die Compliance eines Schlauchs bei Überdruck sehr viel kleiner als bei Unterdruck, so daß das System steifer ist. Da gasfördernde Pumpen eine hohe Förderleistung haben, kann der zu belüftende Teil des Dialysierflüssigkeitssystems schnell mit Gas gefüllt werden. Schon zu Beginn der Messung stellt sich ein hoher Anfangsdruck ein. Dieser beträgt in der Praxis bereits 0,8 bar und steigert sich schnell auf den Meßdruck.

Vorteilhafterweise wird der Überdruck mit einer Luftpumpe aufgebaut, deren Förderrate höher ist als die Förderrate einer im Dialysierflüssigkeitssystem angeordneten Ultrafiltrationspumpe, die bei dem aus DE 34 48 262 C2 bekannten Verfahren zur Erzeugung des Unterdrucks verwendet wird. Der Prüfdruck kann in ca. 10 bis 15 Sekunden aufgebaut werden, so daß sich die gesamte Testdauer mit dem erfindungsgemäßen Verfahren auf ca. 60 Sekunden begrenzen läßt.

Von besonderem Vorteil ist, daß bei dem erfindungsgemäßen Verfahren eine Verfälschung des Ergebnisses der Prüfung durch Nachentgasung nicht auftritt. Obwohl Dialysat selbst entgast ist, kommt es bei einer Überprüfung eines Filters mittels Unterdruck dennoch zu einer Nachentgasung der Lösung, wenn der Unterdruck zu schnell bzw. zu hoch aufgebaut wird. Um dadurch entstehende Probleme bei der Auswertung zu vermeiden, muß der Differenzdruck bei der Unterdruckmessung stark begrenzt werden. Im übrigen dauert es bei einem Unterdrucktest relativ lange, bis der eigentliche Meßdruck erreicht ist. Ferner ist ein Druckhaltetest während des Desinfizierens oder Spülens des Dialysierflüssigkeitssystems nicht möglich, da auch hier eine Nachentgasung der Flüssigkeiten erfolgt. Die Überprüfung der Filter nach dem erfindungsgemäßen Verfahren mittels Überdruck ist hingegen zu jeder Zeit und mit jeder beliebigen Flüssigkeit durchführbar.

Die Zuführung der wässrigen Flüssigkeit zur Benetzung der Membran des zu prüfenden Filters kann mittels der üblicherweise im Dialysierflüssigkeitssystem einer Vorrichtung zur extrakorporalen Blutbehandlung befindlichen Förderpumpen erfolgen. Als Mittel zum druckdichten Absperren des Zweiges des Dialysierflüssigkeitssystems, der eine der beiden Kammern des Filters enthält, finden vorzugsweise elektromagnetisch betätigbare Absperrventile Verwendung. Die Überwachung des Überdrucks erfolgt vorteilhafterweise mit einer Überwachungseinrichtung, die den Wert des Druckabfalls pro Zeiteinheit mit einem vorgegebenen Grenzwert vergleicht und eine Undichtigkeit anzeigt, wenn der vorgegebene Grenzwert überschritten wird.

Im folgenden wird unter Bezugnahme auf die Zeichnungen ein Ausführungsbeispiel der Erfindung näher erläutert.

Es zeigen:
- Fig. 1: eine schematische Darstellung einer Hämo(dia)filtrationsvorrichtung, deren Dialysierflüssigkeitssystem zwei Filter enthält und
- Fig. 2: das Dialysierflüssigkeitssystem der Hämo(dia)filtrationsvorrichtung während der Testphase.

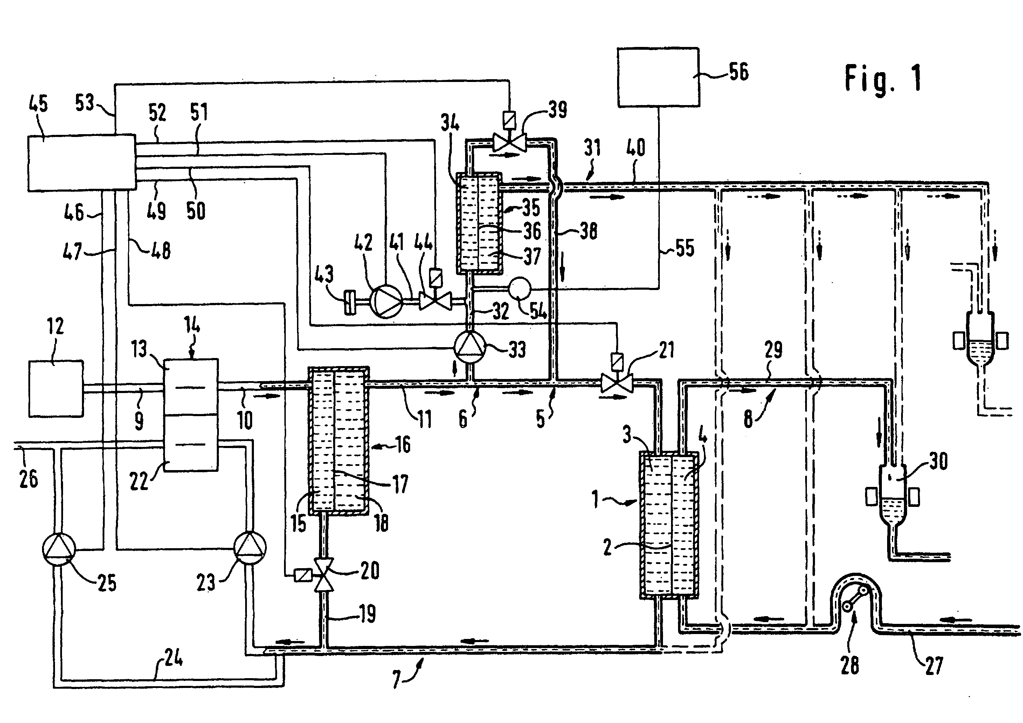

Fig. 1 zeigt eine schematische Darstellung der wesentlichen Komponenten einer Hämo(dia)filtrationsvorrichtung, welche die Durchführung von mehreren Blutbehandlungsverfahren erlaubt. Das Dialysierflüssigkeitssystem der extrakorporalen Blutbehandlungsvorrichtung weist einen Dialysator 1 auf, der durch eine Membran 2 in eine von Dialysierflüssigkeit durchflossene erste Kammer 3 und eine vom Blut durchflossene zweite Kammer 4 getrennt ist.

Die erste Kammer 3 ist in einen Dialysierflüssigkeitsweg 5 des Dialysierflüssigkeitssystems geschaltet, der eine Zuleitung 6 und eine Ableitung 7 aufweist, während die zweite Kammer 4 in einen Blutweg 8 geschaltet ist.

Die Zuleitung 6 des Dialysierflüssigkeitsweges 5 besteht aus einem ersten Zuleitungsabschnitt 9, einem zweiten Zuleitungsabschnitt 10 sowie einem dritten Zuleitungsabschnitt 11 und verbindet eine Dialysierflüssigkeitsquelle 12 mit der ersten Kammer 3 des Dialysators 1.

Der erste Zuleitungsabschnitt 9 verbindet die Dialysierflüssigkeitsquelle 12 mit der ersten Bilanzkammer 13 einer Bilanziervorrichtung 14. Die erste Bilanzkammer 13 der Bilanziervorrichtung 14 ist über den zweiten Zuleitungsabschnitt 10 mit dem Eingang der ersten Kammer 15 eines ersten Filters 16 verbunden, der durch eine Keime zurückhaltende hydrophile Membran 17 in eine erste und zweite Kammer 15, 18 geteilt ist. Der Ausgang der ersten Kammer 15 steht über eine Bypassleitung 19, in die ein elektromagnetisch betätigbares Bypassventil 20 geschaltet ist, mit der Ableitung 7 in Verbindung.

Die zweite Kammer 18 des ersten Filters 16 ist über den dritten Zuleitungsabschnitt 11 mit dem Eingang der ersten Kammer 3 des Dialysators 1 verbunden. In dem dritten Zuleitungsabschnitt 11 ist ein elektromagnetisch betätigbares Dialysator-Ventil 21 geschaltet. Vom Ausgang der ersten Kammer 3 des Dialysators 1 führt die Ableitung 7 zur zweiten Bilanzkammer 22 der Bilanziervorrichtung 14, wobei in die Ableitung eine Dialysierflüssigkeitspumpe 23 geschaltet ist. Stromaufwärts der Dialysierflüssigkeitspumpe 23 geht von der Ableitung 7 eine Ultrafiltratleitung 24 ab, in die eine Ultrafiltrationspumpe 25 zum Abziehen von Dialysierflüssigkeit geschaltet ist. Die Ultrafiltratleitung 24 führt zu einem Abfluß 26, an den auch der Ausgang der zweiten Bilanzkammer 22 der Bilanziervorrichtung 14 angeschlossen ist.

Der Blutweg 8 weist eine vom Patienten kommende Blutzuleitung 27 auf, die an den Eingang der zweiten Kammer 4 des Dialysators 1 angeschlossen ist. In die Blutzuleitung 27 ist eine Pumpe 28 geschaltet. Der Ausgang der zweiten Kammer 4 des Dialysators 1 führt über den ersten Abschnitt einer Blutableitung 29 zu einer Tropfkammer 30, von der Blut über den zweiten Abschnitt der Blutableitung 29 zum Patienten geführt wird.

Das Dialysierflüssigkeitssystem weist ferner einen Substituatkreislauf 31 auf, der von dem dritten Zuleitungsabschnitt 11 des Dialysierflüssigkeitsweges 5 stromauf des Dialysator-Ventils 21 abzweigt und über einen ersten Leitungsabschnitt 32, in welchen eine Substituatpumpe 33 geschaltet ist, zu dem Eingang der ersten Kammer 34 eines zweiten Filters 35 führt, der durch eine Keime zurückhaltende hydrophile Membran 36 in eine erste und zweite Kammer 34, 37 geteilt ist. Von dem Ausgang der ersten Kammer 34 des zweiten Filters 35 geht eine Verbindungsleitung 38 ab, die mit der Zuleitung 6 des Dialysierflüssigkeitsweges 5 stromauf des Dialysator-Ventils 21 verbunden ist. In diese Verbindungsleitung 38 ist ein elektromagnetisch betätigbares Absperrventil 39 geschaltet.

Der zweite Abschnitt 40 des Substituatkreislaufs 31 geht von der zweiten Kammer 37 des zweiten Filters 35 ab und kann wahlweise mit der Blutzuleitung 27 (Prädilution), mit der Tropfkammer 30 (Postdilution) oder zum Spülen des Dialysierflüssigkeitssystems sowie zum Überprüfen der Membranen 17, 36 der im Dialysierflüssigkeitssystem angeordneten Filter mit der Ableitung 7 verbunden werden. Die einzelnen Verbindungszweige sind in Fig. 1 durch gestrichelte Linien angedeutet. Die Blutbehandlungsvorrichtung kann auch ausschließlich als Hämofiltrationsvorrichtung arbeiten. In diesem Fall wird das Dialysator-Ventil 21 geschlossen.

Von dem ersten Leitungsabschnitt 32 des Substituatkreislaufs 31 zweigt eine weitere Leitung 41 ab, die mit dem Luftauslaß einer Luftpumpe 42 verbunden ist. Der Lufteinlaß der Luftpumpe 42 steht über einen hydrophoben Sterilfilter 43 mit der Atmosphäre in Verbindung. In die Leitung 41 ist ein elektromagnetisch betätigbares Absperrventil 44 geschaltet.

Ferner ist eine Steuereinheit 45 vorgesehen, die über Steuerleitungen 46 bis 53 mit der Ultrafiltrationspumpe 25, der Dialysierflüssigkeitspumpe 23, der Substituatpumpe 33, der Luftpumpe 42, dem Bypassventil 20, dem Dialysator-Ventil 21, dem Retentat-Ventil 39 und der im Leitungsabschnitt 41 der Luftpumpe 42 angeordneten Absperrventil 44 verbunden ist. An den ersten Leitungsabschnitt 32 des Substituatkreislaufs 31 ist ein Drucksensor 54 angeschlossen, der über eine Signalleitung 55 mit einer Überwachungseinrichtung 56 verbunden ist.

Nachfolgend wird die Funktionsweise der Hämo(dia)filtrationsvorrichtung beschrieben.

Von der Dialysierflüssigkeitsquelle 12 wird frische Dialysierflüssigkeit über die erste Bilanzkammer 13 der Bilanziervorrichtung 14 und den ersten Filter 16 in die erste Kammer 3 des Dialysators geleitet. Die durch die frische Dialysierflüssigkeit in die Zuleitung 6 eingeschleppten Keime und Pyrogene werden durch die Membran 17 des ersten Filters 16 gefiltert und setzen sich an dieser ab. Durch Öffnen des Bypassventils 20 in der Bypassleitung 19, die eine Verbindung von der ersten Kammer 15 des Filters 16 zur Ableitung 7 herstellt, erfolgt eine Durchspülung der ersten Kammer 15 mit Dialysierflüssigkeit, so daß die an der Membran abgesetzten Keime und Pyrogene durch die Dialysierflüssigkeit mitgerissen und in den Abfluß gespült werden.

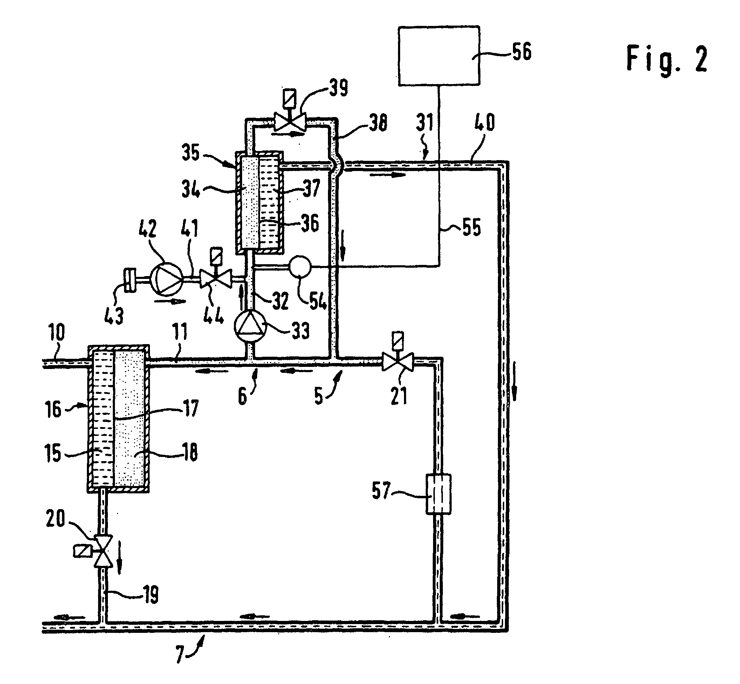

Nach dem Einschalten der Substituatpumpe 33 wird Dialysierflüssigkeit durch die Membran 36 des zweiten Filters 35 der Zuleitung 6 des Dialysierflüssigkeitsweges entzogen und dem Blutkreislauf 8 zugeführt. Das Freispülen der Membran 36 des zweiten Filters 35 erfolgt durch Öffnen des in der Verbindungsleitung 38 zwischen der ersten Kammer 34 des zweiten Filters 35 und der Zuleitung 6 des Dialysierflüssigkeitsweges angeordneten Sperrventils 39. Während des Spülvorgangs wird das Dialysator-Ventil 21 geöffnet und anstelle des Dialysators 1 wird ein Kurzschlußstück 57 an das Schlauchsystem angeschlossen (Fig. 2).

Nachfolgend wird das Verfahren zum Überprüfen der Dichtheit der im Dialysierflüssigkeitssystem 5 angeordneten Filter 16, 35 beschrieben.

Zunächst werden die Leitungen des Dialysierflüssigkeitssystems durch Betätigung der Dialysierflüssigkeitspumpe 23 mit Dialysierflüssigkeit gefüllt, so daß die Membranen 17, 36 des ersten und zweiten Filters 16, 35 völlig mit der wässrigen Lösung benetzt und damit für Gas undurchlässig sind. Die Integritätsprüfung der Membranen 17, 36 kann sich z.B. unmittelbar an den Spülzyklus des Dialysierflüssigkeitssystems anschließen. Fig. 2 zeigt das Dialysierflüssigkeitssystem der Hämo(dia)filtrationsvorrichtung, wobei der Substituatkreislauf 31 mit der Ableitung 7 verbunden und für die Durchführung des Spülzyklus anstelle der ersten Kammer 3 des Dialysators 1 das Kurzschlußstück 57 in den Dialysierflüssigkeitsweg geschaltet ist.

Zum gleichzeitigen Überprüfen der Membranen 17, 36 der beiden Filter 16, 35 auf Lecks wird das Dialysator-Ventil 21 geschlossen und das Bypassventil 20 wird geöffnet. Ferner wird das in der Verbindungsleitung 32 zwischen der ersten Kammer 34 des zweiten Filters 35 und der Zuleitung 6 geschaltete Retentat-Ventil 39 geöffnet und das vor der Luftpumpe 42 angeordnete Sperrventil 44 wird geöffnet. Ferner wird der Substituatkreislauf 31 mit der zum Abfluß 26 führenden Ableitung 7 verbunden. Anschließend wird die Luftpumpe 42 in Betrieb gesetzt, die Luft durch die den hydrophoben Sterilfilter 43 ansaugt und in den Zweig des Dialysierflüssigkeitssystems geleitet, der die zweite Kammer 18 des ersten Filters 16 und die erste Kammer 34 des zweiten Filters 35 enthält. In den beiden Kammern 18, 34 der Filter 16, 35 und den angrenzenden Leitungsabschnitten, d.h. der Verbindungsleitung 32 und dem dritten Leitungsabschnitt 11 der Zuleitung 6 baut sich ein Überdruck auf.

Dabei wird die in der ersten Kammer 34 des zweiten Filters 35 und der zweiten Kammer 18 des ersten Filters 16 sowie in den angrenzenden Leitungsabschnitten befindliche Dialysierflüssigkeit durch die Membranen 17, 36 der beiden Filter 16, 35 verdrängt. Die Dialysierflüssigkeit fließt über die Verbindungsleitung 38 und über die erste Kammer 15 des ersten Filters 16 durch die Bypassleitung 19 in die zum Abfluß 26 führende Ableitung 7 ab und über die zweite Kammer 37 des zweiten Filters und den Abschnitt 40 des Substituatkreislaufs 31 in die zum Abfluß 26 führende Ableitung 7 ab. Sobald in dem abgetrennten Zweig des Dialysierflüssigkeitssystems ein vorbestimmter Überdruck aufgebaut ist, wird die Luftpumpe 42 abgeschaltet.

In der Überwachungseinrichtung 56 wird der mittels des Drucksensors 54 erfaßte Überdruck überwacht und es wird der Druckabfall innerhalb einer vorbestimmten Zeitdauer ermittelt. Anschließend wird der Druckabfall pro Zeiteinheit mit einem vorgegebenen Grenzwert verglichen, der für den Druckabfall pro Zeiteinheit einer intakten Filtermembran charakteristisch ist. Sofern der Druckabfall innerhalb der vorbestimmten Zeitdauer größer als der Grenzwert ist, gibt die Überwachungseinrichtung einen akustischen und/oder optischen Alarm. In diesem Fall ist die eine und/oder andere Filtermembran 17, 36 undicht. Alternativ ist es aber auch möglich, nur die Membran eines Filters oder mehrerer Filter mit dem erfindungsgemäßen Verfahren zu überprüfen. Hierzu sind in den Leitungsabschnitten entsprechende Absperrorgane vorzusehen, die den Aufbau eines Überdrucks nur in der Kammer des zu überprüfenden Filters erlauben. Theoretisch kann auch der Dialysator selbst so geprüft werden, doch sollte dies nie mit Patienten an der Blutleitung geschehen.

Die Ansteuerung der elektromagnetisch betätigbaren Ventile 20, 21, 39 und 44 und die Inbetriebnahme der Luftpumpe 42 erfolgt nach einem vorgegebenen Programm vollautomatisch durch die in Fig. 1 dargestellte Steuereinheit 45 der Hämo(dia)filtrationsvorrichtung.

## Patentansprüche

1. Verfahren zum Überprüfen von mindestens einem im Dialysierflüssigkeitssystem einer Vorrichtung zur extrakorporalen Blutbehandlung angeordneten Filter (16, 35), der durch eine Keime zurückhaltende Membran (17, 36) in eine erste Kammer (15, 34) und eine zweite Kammer (18, 37) geteilt ist, wobei
zumindest ein Zweig des Dialysierflüssigkeitssystems unter völliger Benetzung der Membran (17, 36) des zu prüfenden Filters bzw. der Filter (16, 35) mit einer wässrigen Flüssigkeit gefüllt wird,
ein Zweig des Dialysierflüssigkeitssystems, der eine der beiden Kammern (18, 37) des Filters bzw. der Filter (16, 35) enthält, von den übrigen Zweigen des Dialysierflüssigkeitssystems abgesperrt wird,
**dadurch gekennzeichnet,**
**dass** in den die eine Kammer (18, 37) des Filters bzw. der Filter (16, 35) enthaltenden Zweig des Dialysierflüssigkeitssystems unter Verdrängung der Flüssigkeit durch die Membran (17, 36) des Filters bzw. der Filter (16, 35) zum Aufbau eines Überdrucks Gas geleitet wird,
**dass** nach Erreichen des vorbestimmten Überdrucks in der einen Kammer (18, 34) des Filters bzw. der Filter (16, 35) die Gaszufuhr abgeschaltet wird und
**dass** der Überdruck in der einen Kammer (18, 34) des Filters bzw. der Filter (16, 35) überwacht wird.

2. Verfahren nach Anspruch 1,
**dadurch gekennzeichnet, dass** die wässrige Flüssigkeit zur Benetzung der Membran (17, 36) des zu prüfenden Filters bzw. der Filter (16, 35) Dialysierflüssigkeit ist.

3. Verfahren nach Anspruch 1 oder 2,
**dadurch gekennzeichnet, dass** als Gas Luft aus der Atmosphäre über mindestens ein hydrophobes Filter (43) in den die eine Kammer (18, 34) des Filters bzw. der Filter (16, 35) enthaltenden Zweig des Dialysierflüssigkeitssystems geleitet wird.

4. Verfahren nach einem der Ansprüche 1 bis 3,
**dadurch gekennzeichnet, dass** der Druckabfall pro Zeiteinheit mit einem vorgegebenen Grenzwert verglichen und bei Überschreiten des Grenzwertes auf eine Undichtigkeit geschlossen wird.

5. Dialysierflüssigkeitssystem einer Vorrichtung zur extrakorporalen Blutbehandlung, das mindestens einen Filter (16, 35) enthält, der durch eine Keime zurückhaltende Membran (17, 36) in eine erste Kammer (15, 34) und eine zweite Kammer (18, 37) geteilt ist, wobei das Dialysierflüssigkeitssystem umfaßt
Mittel (23) zum Zuführen einer wässrigen Flüssigkeit in zumindest einen Zweig des Dialysierflüssigkeitssystems unter völliger Benetzung der Membran (17, 36) des zu prüfenden Filters bzw. der Filter (16, 35),
Mittel (21) zum Absperren eines Zweiges des Dialysierflüssigkeitssystems, der eine der beiden Kammern (18, 37) des Filters bzw. der Filter (16, 35) enthält, von den übrigen Zweigen des Dialysierflüssigkeitssystems,
**gekennzeichnet durch**
Mittel (42) zum Zuführen eines Gases in den die eine Kammer (18, 34) des Filters bzw. der Filter (16, 35) enthaltenden Zweig des Dialysierflüssigkeitssystems unter Verdrängung der Flüssigkeit **durch** die Membran (17, 36) des Filters bzw. der Filter (16, 35) zum Aufbau eines Überdrucks und
eine Überwachungseinrichtung (56) zum Überwachen des Überdruckes in der einen Kammer (18, 34) des Filters bzw. der Filter (16, 35).

6. Dialysierflüssigkeitssystem nach Anspruch 5,
**dadurch gekennzeichnet, dass** das Mittel zum Zuführen des Gases eine Luft aus der Atmosphäre über mindestens ein hydrophobes Filter (43) ansaugende Luftpumpe (42) ist.

7. Dialysierflüssigkeitssystem nach Anspruch 5 oder 6,
**dadurch gekennzeichnet, dass** die Überwachungseinrichtung (56) den Wert des Druckabfalls pro Zeiteinheit mit einem vorgegebenen Grenzwert vergleicht und eine Undichtigkeit anzeigt, wenn der vorgegebene Grenzwert überschritten wird.

## Claims

1. Method for testing at least one filter(16, 35) arranged in the dialysing fluid system of an apparatus for extracorporeal blood treatment, which filter is divided by a membrane (17, 36) retaining bacteria into a first chamber (15, 34) and a second chamber (18, 37), wherein
at least one branch of the dialysing fluid system is filled with an aqueous fluid with full moistening of the membrane (17, 36) of the filter or filters (16, 35) to be tested,
one branch of the dialysing fluid system which contains one of the two chambers (18, 37) of the filter or filters (16, 35) is blocked by the other branches of the dialysing fluid system,
**characterised**
**in that** gas is conducted into the branch of the dialysing fluid system containing one chamber (18, 37) of the filter or filters (16, 35), the fluid being displaced through the membrane (17, 36) of the filter or filters (16, 35) in order to develop an excess pressure,
**in that** the gas supply is switched off after the predetermined excess pressure is reached in one of the chambers (18, 34) of the filter or filters (16, 35), and
**in that** the excess pressure in one of the chambers (18, 34) of the filter or filters (16, 35) is monitored.

2. Method according to claim 1, **characterised in that** the aqueous fluid for moistening the membrane (17, 36) of the filter or filters (16, 35) to be tested is dialysing fluid.

3. Method according to claim 1 or 2, **characterised in that** air from the atmosphere is conducted as gas through at least one hydrophobic filter (43) into the branch of the dialysing fluid system containing one chamber (18, 34) of the filter or filters (16, 35).

4. Method according to one of claims 1 to 3, **characterised in that** the reduction in pressure per unit of time is compared with a predetermined limit value and if the limit value is exceeded this indicates a leak.

5. Dialysing fluid system of an apparatus for extracorporeal blood treatment, which contains at least one fitter (16, 35) which is divided by a membrane (17, 36) retaining bacteria into a first chamber (15, 34) and a second chamber (18, 37), wherein the dialysing fluid system contains
means (23) for feeding an aqueous fluid into at least one branch of the dialysing fluid system, with full moistening of the membrane (17, 36) of the filter or filters (16, 35) to be tested,
means (21) for shutting off one branch of the dialysing fluid system containing one of the two chambers (18, 37) of the filter or filters (16, 35) from the other branches of the dialysing fluid system,
**characterised by**
means (42) for feeding a gas into the branch of the dialysing fluid system containing one chamber (18, 34) of the filter or filters (16, 35), the fluid being displaced through the membrane (17, 36) of the filter or filters (16, 35) for developing an excess pressure, and
a monitoring device (56) for monitoring the excess pressure in one chamber (18, 34) of the filter or filters (16, 35).

6. Dialysing fluid system according to claim 5,
**characterised in that** the means for feeding the gas is an air pump (42) sucking air from the atmosphere through at least one hydrophobic filter (43).

7. Dialysing fluid system according to claim 5 or 6,
**characterised in that** the monitoring device (56) compares the value of the reduction in pressure per unit of time with a predetermine limit value and indicates a leak if the predetermined limit value is exceeded.

## Revendications

1. Procédé pour tester au moins un filtre (16,35) monté dans le circuit de liquide de dialyse d'un dispositif pour le traitement extracorporel du sang qui est divisé en une première chambre (15, 34) et une seconde chambre (18, 37) par une membrane (17, 36) retenant les germes, moyennant quoi au moins une branche du circuit de liquide de dialyse est remplie avec un liquide aqueux, avec un mouillage total de la membrane (17, 36) du filtre à tester ou des filtres (16, 35), une branche du circuit de liquide de dialyse, qui comprend l'une des deux chambres (18, 37) du filtre ou des filtres, est fermée par rapport aux autres branches du circuit de liquide de dialyse, **caractérisé en ce que**, dans la branche du circuit de liquide de dialyse contenant une chambre (18, 37) du filtre ou des filtres (16, 35), du gaz est guidé par déplacement du liquide à travers la membrane (17, 36) du filtre ou des filtres (16, 35) pour établir une surpression, **en ce que**, après obtention de la surpression prédéterminée dans la chambre (18, 34) du filtre ou des filtres (16, 35), l'alimentation en gaz est arrêtée et **en ce que** la surpression dans la chambre (18, 34) du filtre ou des filtres (16, 35) est surveillée.

2. Procédé selon la revendication 1, **caractérisé en ce que** le liquide aqueux pour le mouillage de la membrane (17, 36) du filtre à tester ou des filtres (16, 35) est un liquide de dialyse.

3. Procédé selon la revendication 1 ou 2, **caractérisé en ce que** le gaz est de l'air qui est guidé à partir de l'atmosphère par l'intermédiaire d'au moins un filtre hydrophobe (43) dans la branche du circuit de liquide de dialyse contenant la chambre (18, 34) du filtre ou des filtres (16, 35).

4. Procédé selon l'une quelconque des revendications 1 à 3, **caractérisé en ce que** la chute de pression par unité de temps est comparée à une valeur limite prédéterminée et, en cas de dépassement de la valeur limite, on conclut à un défaut d'étanchéité.

5. Circuit de liquide de dialyse d'un dispositif pour le traitement extracorporel du sang qui comprend au moins un filtre (16, 35) qui est divisé en une première chambre (15, 34) et une seconde chambre (18, 37) par une membrane (17, 36) retenant les germes, moyennant quoi le circuit de liquide de dialyse comprend un moyen (23) pour alimenter un liquide aqueux dans au moins une branche du circuit de liquide de dialyse avec un mouillage total de la membrane (17, 36) du filtre à tester ou des filtres (16, 35), un moyen (21) pour fermer une branche du circuit de liquide de dialyse, qui contient l'une des deux chambres (18, 37) du filtre ou des filtres (16, 35), par rapport aux autres branches du circuit de liquide de dialyse, **caractérisé par** un moyen (42) pour alimenter un gaz dans la branche du circuit de liquide de dialyse contenant la chambre (18, 34) du filtre ou des filtres (16, 35) par déplacement du liquide à travers la membrane (17, 36) du filtre ou des filtres (16, 35) pour établir une surpression et un dispositif de surveillance (56) pour surveiller la surpression dans la chambre (18, 34) du filtre ou des filtres (16, 35).

6. Circuit de liquide de dialyse selon la revendication 5, **caractérisé en ce que** le moyen pour l'alimentation du gaz est une pompe à air (42) aspirant l'air provenant de l'atmosphère par l'intermédiaire d'au moins un filtre hydrophobe (43).

7. Circuit de liquide de dialyse selon la revendication 5 ou 6, **caractérisé en ce que** le dispositif de surveillance (56) compare la valeur de la chute de pression par unité de temps à une valeur limite prédéterminée et indique un défaut d'étanchéité lorsque la valeur limite prédéterminée est dépassée.
